# EUROPEAN PATENT APPLICATION

(11) **EP 4 706 692 A2**
(43) Date of publication of application: **11.03.2026**
(21) Application number: 25189372.3
(22) Date of filing: 14.07.2025
(51) Int. Cl.: A61L 2/10, C02F 1/32

(54) **FLUID STERILIZATION DEVICE WITH SIDE LIGHT-EMITTING MODULE**

(30) Priority: 10.09.2024 TW 113134180
(71) Applicant: Hergy International Corp., Taipei City 112 (TW)
(72) Inventor: LUO, Sheng-Tay, 112 Taipei City (TW); LIN, Chia-Te, 112 Taipei City (TW); GUO, Dong-Hong, 112 Taipei City (TW)
(74) Representative: 2K Patentanwälte Blasberg Kewitz & Reichel

(57) **Abstract**

A fluid sterilization device (10) with a side light-emitting module (2) is provided for sterilizing a liquid. The fluid sterilization device (10) includes a main body (1) and one or more side light-emitting modules (2). The main body (1) includes an outer tube (11) and an inner tube (12) placed within the outer tube (11), forming a gap channel (S) between them. The outer tube (11) has hollow tubes (111) arranged corresponding to the gap channel (S). An axial direction of the outer tube (11) is either perpendicular or inclined to an axial direction of each hollow tube (111). The side light-emitting module (2) is inserted into one of the hollow tubes (111) and includes at least one ultraviolet light source (21) for irradiating the gap channel (S). The installed number of side light-emitting modules (2) may be adjusted according to the length of the gap channel (S) or the sterilization efficiency required by the client.

## Description

### BACKGROUND OF THE DISCLOSURE

### Technical Field

The present disclosure relates to a fluid sterilization device, and more particularly, to a fluid sterilization device with a side light-emitting module.

### Description of Related Art

In the market, setups that use ultraviolet light (such as UV lamps or LEDs) for fluid sterilization typically involve positioning the ultraviolet light inside or outside a chamber. These systems utilize a single flow channel design to expose the fluid to ultraviolet light for a specified duration, effectively achieving sterilization.

However, in ultraviolet fluid sterilization devices, the LED lights are crucial for generating the necessary ultraviolet light. The higher the intensity of ultraviolet light that is received at a given volumetric flow rate, the greater the sterilization efficiency. Therefore, a key area of research and development among fluid sterilization device manufacturers is how to modularize the LED lights and adjust the number of LED modules according to different flow channel lengths to meet client-specific requirements for fluid storage capacity and sterilization efficiency.

In view of this, the inventor of the present disclosure has conducted in-depth research and applied scientific principles to diligently address the aforementioned issues in the related art, which has become the objective of the development.

### SUMMARY OF THE DISCLOSURE

The present disclosure provides a fluid sterilization device equipped with a side light-emitting module. This fluid sterilization device features a plurality of hollow tubes extending from an outer tube and aligned with the gap channel. The number of side light-emitting modules may be adjusted by inserting them into the hollow tubes. This arrangement allows the fluid sterilization device to vary the number of side light-emitting modules based on a length of the gap channel S or the sterilization efficiency required by the client.

In one embodiment of the present disclosure, a fluid sterilization device with a side light-emitting module is provided for sterilizing a liquid. The fluid sterilization device includes: a main body including an outer tube and an inner tube disposed within the outer tube, wherein a gap channel is defined between the outer tube and the inner tube, the outer tube has a plurality of hollow tubes, the hollow tubes are arranged corresponding to the gap channel, and an axial direction of the outer tube is perpendicular or inclined to an axial direction of each of the hollow tubes; and at least one side light-emitting module inserted into at least one of the hollow tubes, wherein the side light-emitting module includes at least one ultraviolet light source for irradiating the gap channel, wherein the liquid is irradiated by the at least one ultraviolet light source when the liquid passes through the gap channel.

Based on the above, the outer tube features multiple hollow tubes extending therefrom and arranged corresponding to the gap channel. The axial direction of the outer tube is perpendicular or inclined to the axial direction of each hollow tube. The number of side light-emitting modules installed in the hollow tubes may be adjusted according to a length of the gap channel or the sterilization efficiency required by the client, so that the liquid passing through the gap channel is uniformly irradiated by the ultraviolet light source of the side light-emitting module. This configuration allows the fluid sterilization device of the present disclosure to increase or decrease the number of installed side light-emitting modules based on the length of the gap channel or the sterilization efficiency required by the client, providing the fluid sterilization device with desirable customization capabilities.

### BRIEF DESCRIPTION OF THE DRAWINGS

FIG. 1 is a perspective assembly view of a fluid sterilization device according to the present application.
FIG. 2 is another perspective assembly view of the fluid sterilization device according to the present application.
FIG. 3 is a perspective assembly view of a first cover plate and an inner tube according to the present application.
FIG. 4 is an exploded perspective view of the first cover plate and the inner tube according to the present application.
FIG. 5 is a cross-sectional view of the fluid sterilization device in use according to the present application.
FIG. 6 is a schematic cross-sectional view of the fluid sterilization device according to the present application.
FIG. 7 is another schematic cross-sectional view of the fluid sterilization device according to the present application.
FIG. 8 is a front schematic view of the fluid sterilization device according to another embodiment of the present application.
FIG. 9 is a front schematic view of the fluid sterilization device according to yet another embodiment of the present application.
FIG. 10 is a front view of the fluid sterilization device in use according to still yet another embodiment of the present application.

### DETAILED DESCRIPTION

The following detailed descriptions of the present application, along with the accompanying drawings, illustrate the technical features of the application. However, the accompanying drawings are for illustrative purposes only and are not intended to limit the scope of the application.

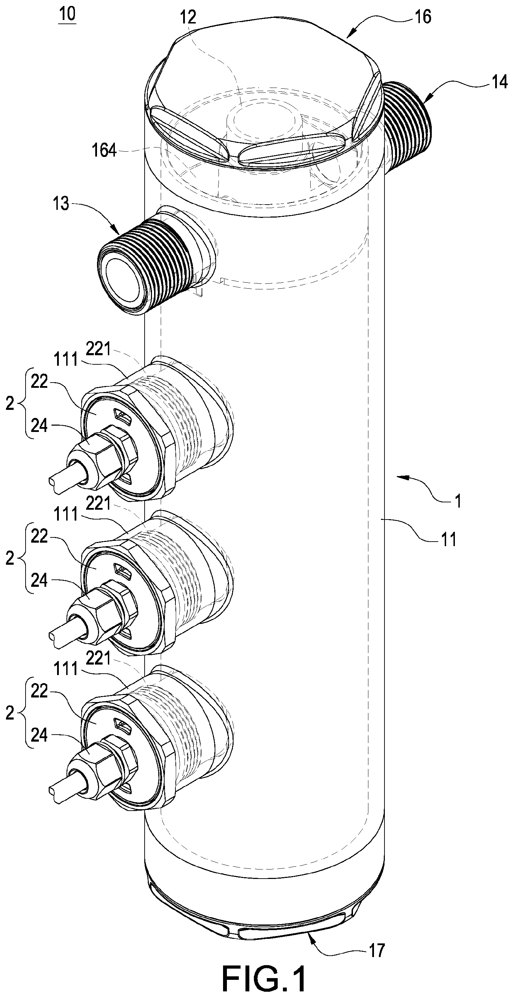

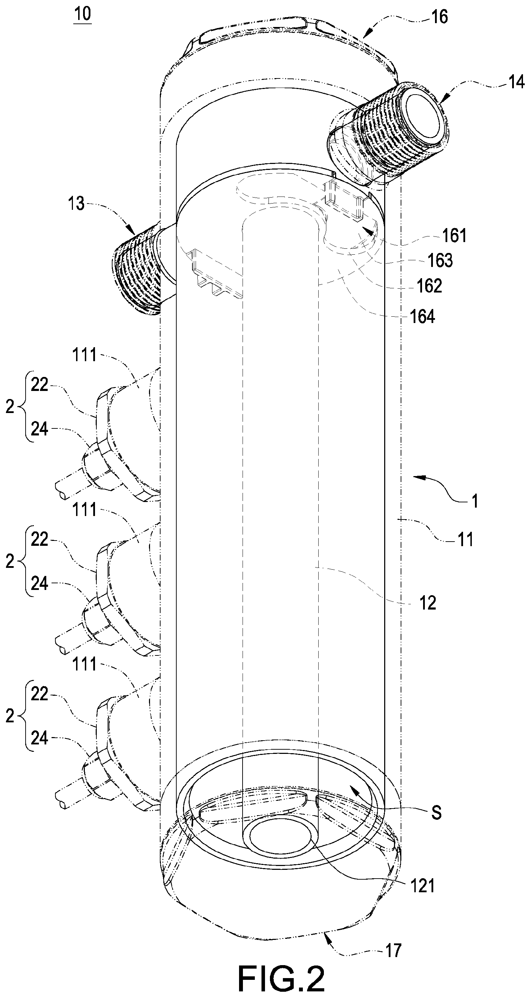

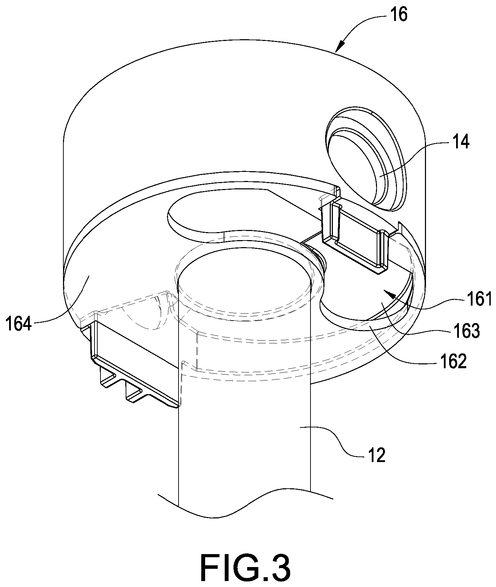

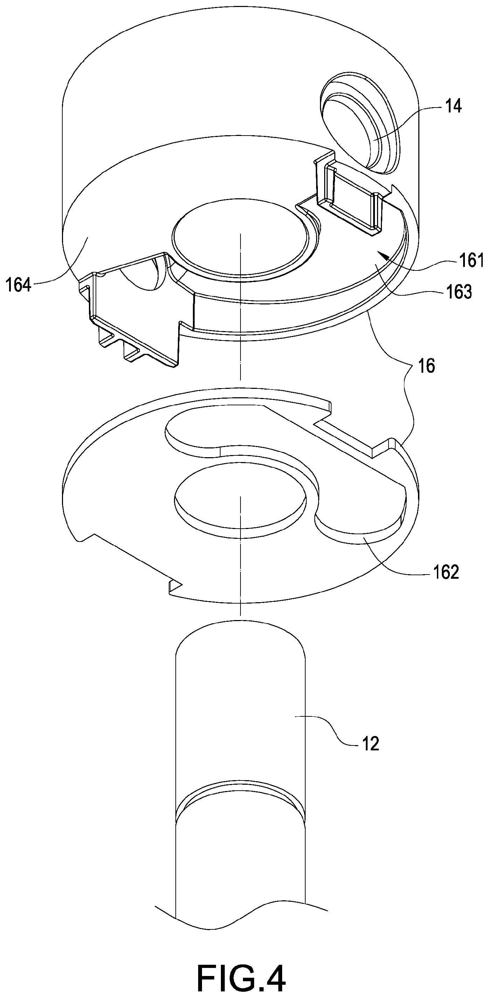

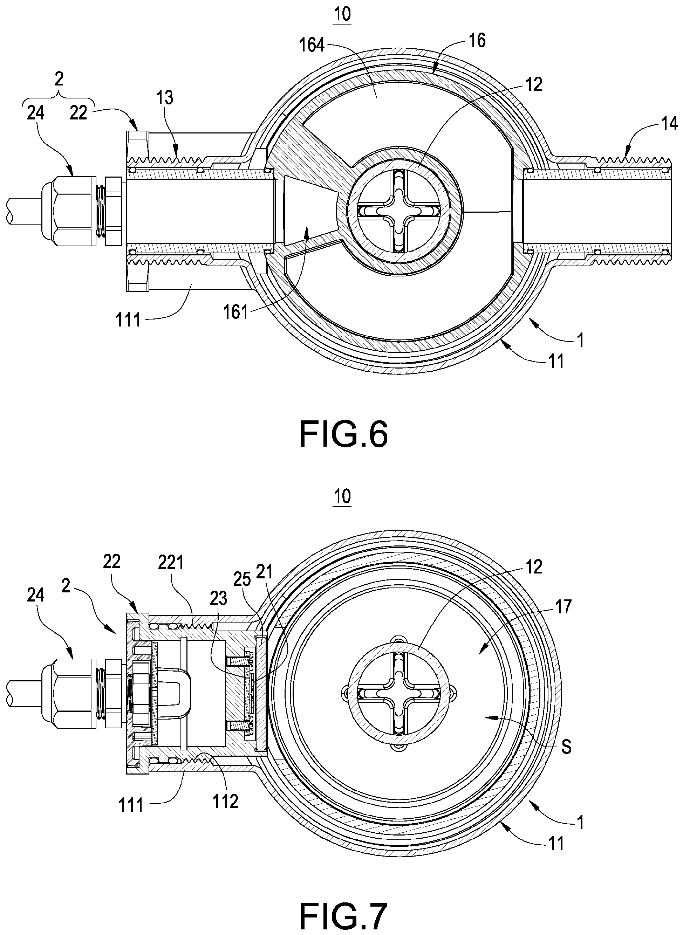

Referring to FIGS. 1 to 7, the present application provides a fluid sterilization device with a side light-emitting module for sterilizing liquids such as water (not shown in the drawings). The fluid sterilization device 10 mainly includes a main body 1 and one or more side light-emitting modules 2.

As shown in FIGS. 1 to 7, the main body 1 includes an outer tube 11 and an inner tube 12 disposed within the outer tube 11. A gap channel S is formed between the outer tube 11 and the inner tube 12. The outer tube 11 includes a plurality of hollow tubes 111 extending therefrom. The hollow tubes 111 are arranged corresponding to the gap channel S. Each hollow tube 111 is provided with an internal thread 112.

In detail, in this embodiment, an axial direction D1 of the outer tube 11 is perpendicular to an axial direction D2 of each hollow tube 111. However, this configuration is not restrictive; the axial direction D1 of the outer tube 11 may also be inclined relative to the axial direction D2 of each hollow tube 111. It is only specified that the axial direction D1 of the outer tube 11 should not be parallel to the axial direction D2 of each hollow tube 111.

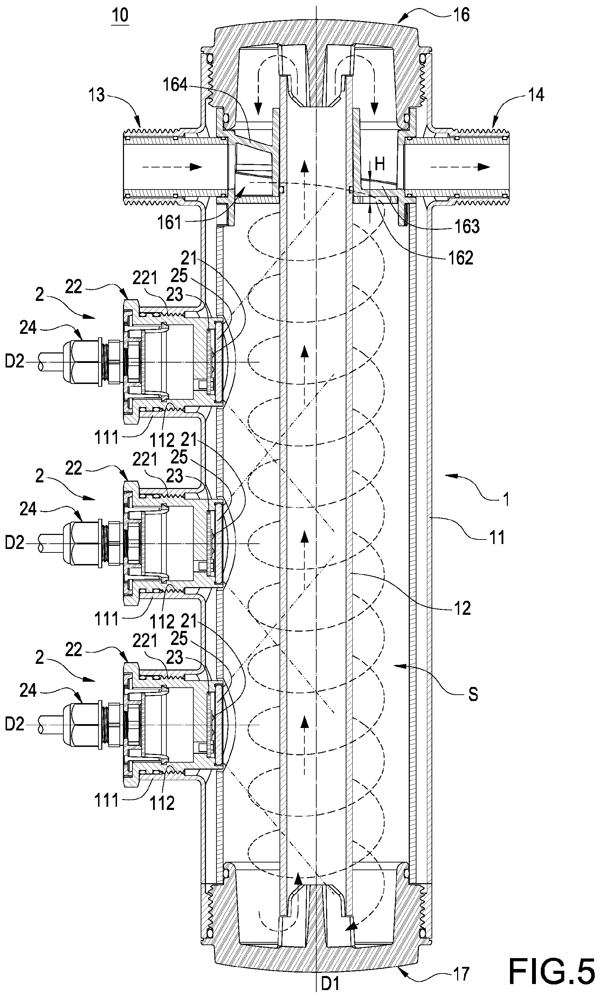

Furthermore, in this embodiment, the hollow tubes 111 are arranged spaced apart and side-by-side in an up-down direction on one side of the outer tube 11. The outer tube 11 is made of a reflective material such as stainless steel, and the inner tube 12 is made of a light-transmissive material such as glass. The main body 1 further includes a liquid inlet tube 13 and a liquid outlet tube 14. One end of the inner tube 12 is connected only to the liquid outlet tube 14, and the other end of the inner tube 12 is provided with a communication port 121. One end of the outer tube 11 only communicates to the liquid inlet tube 13, and the other end of the outer tube 11 is spaced apart from the communication port 121. As shown by the arrow direction in FIG. 5, the liquid sequentially flows through the liquid inlet tube 13, the gap channel S, the communication port 121, the inner tube 12, and exits through the liquid outlet tube 14.

Furthermore, in this embodiment, portions of the liquid inlet tube 13 and the liquid outlet tube 14 each extend from two sides of one end of the outer tube 11. As depicted in FIG. 5, portions of the liquid inlet tube 13 extend from a left side, and the liquid outlet tube 14 extends from a right side of the upper end of the outer tube 11.

Furthermore, in this embodiment, the main body 1 further includes a first cover plate 16 and a second cover plate 17 that cover and seal both ends of the outer tube 11. The first cover plate 16 is structured by a cover body and a separate plate arranged as separate parts, but the present disclosure is not limited to this configuration. The first cover plate 16 includes a helical guide path 161, another portion of the liquid outlet tube 14, and a separator 164. One end of the helical guide path 161 is arranged corresponding to the liquid inlet tube 13, and the other end of the helical guide path 161 is arranged corresponding to the gap channel S. The helical guide path 161 includes an opening 162 communicating to the gap channel S and a flow guide slope 163 that gradually narrows a distance H with respect to the opening 162 in a direction away from the liquid inlet tube 13. The separator 164 is positioned between the helical guide path 161 and the another portion of the liquid outlet tube 14, isolating them from each other and preventing communication. This ensures that the liquid sequentially flows through the liquid inlet tube 13, the helical guide path 161, the gap channel S, the communication port 121, the inner tube 12, and finally to the liquid outlet tube 14. As shown in FIG. 5, the helical guide path 161 guides the liquid to flow downwards in a spiral manner into the gap channel S.

As shown in FIGS. 1 to 2 and 5 to 7, this embodiment features multiple side light-emitting modules 2, but the present disclosure is not limited in this regard. The present disclosure may have just one side light-emitting module 2. Each side light-emitting module 2 is selectively inserted into any of the hollow tubes 111. The side light-emitting module 2 includes one or more ultraviolet light sources 21 for irradiating the gap channel S. Each ultraviolet light source 21 may be a UV-C light-emitting diode (LED), but this configuration is not restrictive, ensuring that the liquid passing through the gap channel S is irradiated by the ultraviolet light source 21.

In this embodiment, the outer tube 11 is made of a reflective material such as stainless steel, allowing the ultraviolet light generated by the ultraviolet light source 21 to scatter off the surface of the outer tube 11. This scatters the light uniformly to irradiate and sterilize the liquid in the gap channel S. The inner tube 12 is made of a light-transmissive material such as glass, so the ultraviolet light generated by the ultraviolet light source 21 is also refracted through the inner tube 12 to the inside of the inner tube 12, thereby uniformly irradiating and sterilizing the liquid inside the inner tube 12.

Further explanation is as follows. The side light-emitting module 2 further includes a housing 22, a circuit board 23 fixedly connected to the housing 22, and a power connector 24. The housing 22 is inserted into one of the hollow tubes 111. In this embodiment, the housing 22 is provided with an external thread 221 that is threadedly engaged with the internal thread 112, so that the housing 22 is securely screwed or unscrewed from the hollow tube 111; however, the present disclosure is not limited in this regard.

Furthermore, in this embodiment, each side light-emitting module 2 contains multiple ultraviolet light sources 21, although the configuration is not limited to this arrangement. These ultraviolet light sources 21 are mounted on the circuit board 23. The circuit board 23 is electrically connected to the power connector 24. This connection allows an external power source to supply power through the power connector 24 to both the circuit board 23 and the ultraviolet light sources 21.

Please refer to FIG. 5, which shows the fluid sterilization device 10 of the present disclosure is in use. The fluid sterilization device 10 utilizes multiple hollow tubes 111 which are extending from the outer tube 11 and arranged corresponding to the gap channel S. The axial direction D1 of the outer tube 11 is perpendicular or inclined to the axial direction D2 of each hollow tube 111. The number of side light-emitting modules 2 installed in the hollow tubes 111 may be adjusted according to a length of the gap channel S or the sterilization efficiency required by the client, so that the liquid passing through the gap channel S is uniformly irradiated by the ultraviolet light source 21 of the side light-emitting module 2. This configuration allows the fluid sterilization device 10 of the present disclosure to increase or decrease the number of installed side light-emitting modules 2 based on the length of the gap channel S or the sterilization efficiency required by the client, providing the fluid sterilization device 10 with excellent customization capabilities.

In addition, the first cover plate 16 and the second cover plate 17 may be assembled or disassembled independently from the main body 1. When the first cover plate 16 and the second cover plate 17 are disassembled from the main body 1, the outer tube 11 and the inner tube 12 are exposed from the main body 1, which facilitates the cleaning of the fluid sterilization device 10 of the present disclosure.

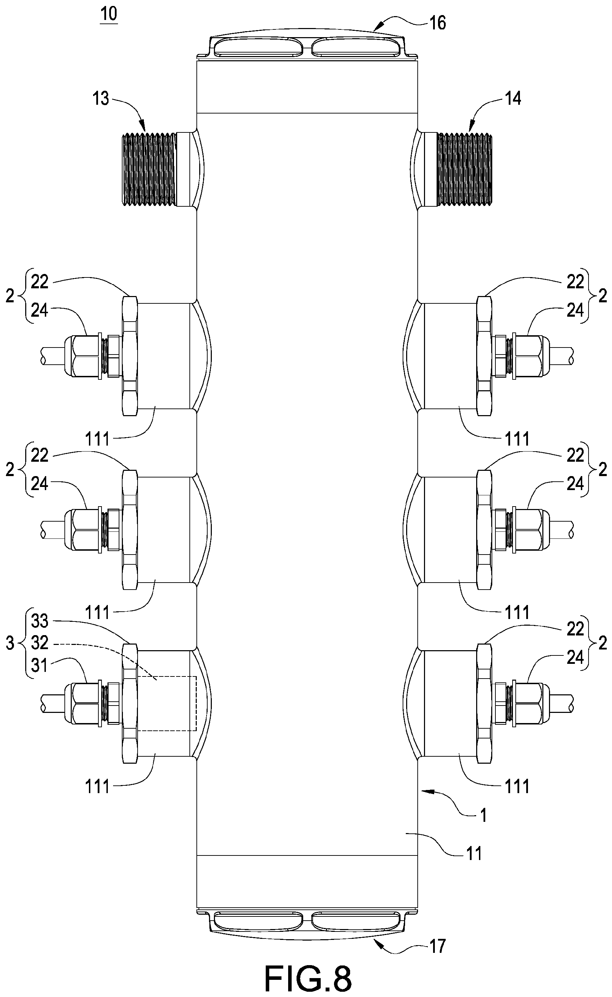

As shown in FIG. 8, this is another embodiment of the fluid sterilization device 10 of the present disclosure. The embodiment of FIG. 8 is generally the same as the embodiment of FIGS. 1 to 7, except that the hollow tubes 111 are arranged spaced apart and side-by-side in the up-down direction on both sides of the outer tube 11, and the fluid sterilization device 10 further includes a water quality sensing module 3.

As shown in FIG. 8, the hollow tubes 111 are arranged spaced apart and side-by-side in the up-down direction on the left and right sides of the outer tube 11, thereby increasing the number of side light-emitting modules 2 that may be installed. The hollow tubes 111 on one side of the outer tube 11 are aligned with the hollow tubes 111 on the other side of the outer tube 11.

In addition, the water quality sensing module 3 may be selectively inserted into one of the remaining hollow tubes 111, that is, one of the hollow tubes 111 is inserted with the water quality sensing module 3 and the other hollow tubes 111 are inserted with the side light-emitting modules 2. Thus, the hollow tubes 111 not only accommodate the side light-emitting modules 2 but also optionally the water quality sensing module 3, enhancing the operational convenience of the fluid sterilization device 10.

The detailed description is as follows. The water quality sensing module 3 includes an outer casing 31, and a water quality sensor 32 and a signal connector 33 fixedly connected to the outer casing 31. The outer casing 31 is inserted into one of the hollow tubes 111. In this embodiment, the outer casing 31 is provided with an external thread that is threadedly engaged with the internal thread 112, so that the outer casing 31 may be securely screwed or unscrewed from the hollow tube 111, but this does not impose a limitation. The water quality sensor 32 is electrically connected to the signal connector 33, so that an external device may receive water quality signals sensed by the water quality sensor 32 through the signal connector 33.

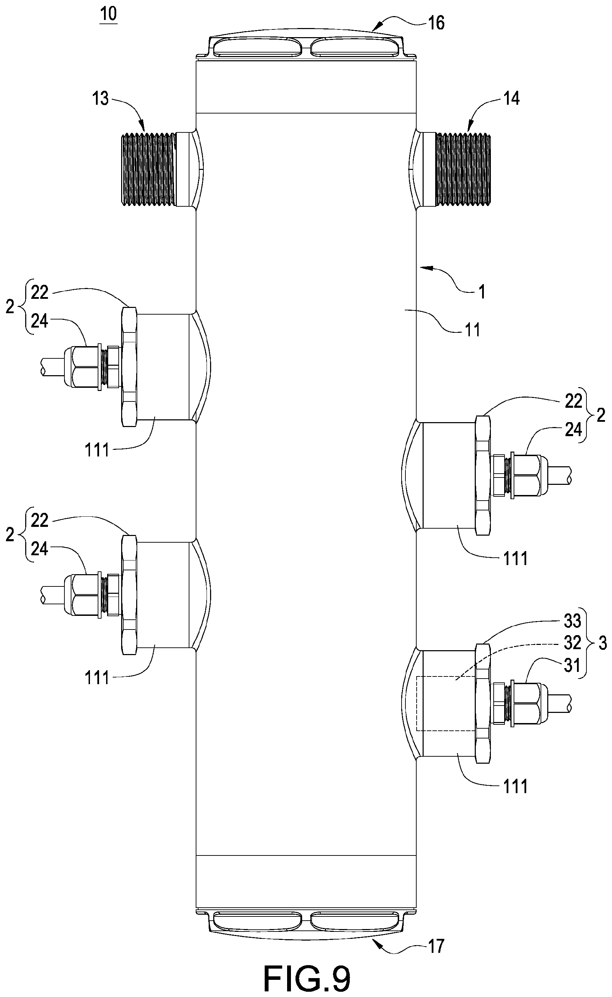

As shown in FIG. 9, this is yet another embodiment of the fluid sterilization device 10 of the present disclosure. The embodiment of FIG. 9 is generally the same as the embodiment of FIG. 8, except that the hollow tubes 111 on one side of the outer tube 11 are staggered with the hollow tubes 111 on the other side of the outer tube 11.

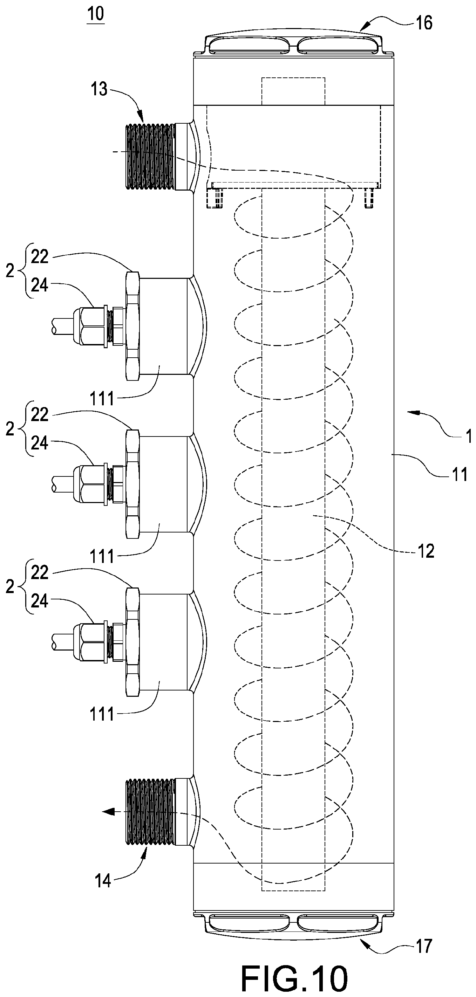

As shown in FIG. 10, this is still yet another embodiment of the fluid sterilization device 10 of the present disclosure. The embodiment of FIG. 10 is generally the same as the embodiment of FIGS. 1 to 7, except that the structures of the outer tube 11 and the inner tube 12 are slightly different.

Further explanation is as follows. In this embodiment, the outer tube 11 and the inner tube 12 are respectively made of a reflective material such as stainless steel, so the ultraviolet light generated by the ultraviolet light source 21 may be scattered off the surfaces of the outer tube 11 and the inner tube 12, thereby uniformly irradiating and sterilizing the liquid in the gap channel S. The main body 1 further includes a liquid inlet tube 13 and a liquid outlet tube 14. One end of the outer tube 11 is connected only to the liquid inlet tube 13, and the other end of the outer tube 11 is connected only to the liquid outlet tube 14. As a result, as shown by the arrow direction in FIG. 10, the liquid sequentially flows through the liquid inlet tube 13, the gap channel S, and finally to the liquid outlet tube 14.

Furthermore, in this embodiment, the liquid inlet tube 13 and the liquid outlet tube 14 extend from two ends of the outer tube 11, respectively. The first cover plate 16 only has a helical guide path 161. One end of the helical guide path 161 corresponds to the liquid inlet tube 13, and the other end of helical guide path 161 corresponds to the gap channel S, as shown in FIG. 5. Notably, the first cover plate 16 in this embodiment does not include the separator 164, as it is not required.

## Claims

1. A fluid sterilization device (10), used for a liquid, the fluid sterilization device (10) comprising:
a main body (1), comprising an outer tube (11) and an inner tube (12) inserted in the outer tube (11), wherein a gap channel (S) is defined between the outer tube (11) and the inner tube (12), the outer tube (11) comprises a plurality of hollow tubes (111) arranged corresponding to the gap channel (S), and an axial direction of the outer tube (11) is perpendicular or inclined to an axial direction of each of the hollow tubes (111); and
at least one side light-emitting module (2), inserted in at least one of the hollow tubes (111), and comprising at least one ultraviolet light source (21) irradiating the gap channel (S);
wherein the liquid is irradiated by the at least one ultraviolet light source (21) when the liquid passes through the gap channel (S).

2. The fluid sterilization device (10) according to claim 1, further comprising a water quality sensing module (3) inserted in one of the hollow tubes (111).

3. The fluid sterilization device (10) according to claim 2, wherein the water quality sensing module (3) comprises an outer casing (31), a water quality sensor (32) and a signal connector (33) both fixedly connected to the outer casing (31), the water quality sensor (32) and the signal connector (33) are electrically connected, and the outer casing (31) is inserted in one of the hollow tubes (111).

4. The fluid sterilization device (10) according to claim 1, wherein the side light-emitting module (2) comprises a housing (22), a circuit board (23) and a power connector (24) both fixedly connected to the housing (22), the at least one ultraviolet light source (21) is mounted on the circuit board (23), the circuit board (23) and the power connector (24) are electrically connected, and the housing (22) is inserted in one of the hollow tubes (111).

5. The fluid sterilization device (10) according to claim 1, wherein the hollow tubes (111) are arranged spaced apart and side-by-side in an up-down direction on one side of the outer tube (11).

6. The fluid sterilization device (10) according to claim 1, wherein the hollow tubes (111) are arranged spaced apart and side-by-side in an up-down direction on two sides of the outer tube (11) opposite to each other.

7. The fluid sterilization device (10) according to claim 1, wherein the outer tube (11) is made of a reflective material, the inner tube (12) is made of a light-transmissive material, the main body (1) further comprises a liquid inlet tube (13) and a liquid outlet tube (14), one end of the inner tube (12) only communicates to the liquid outlet tube (14) and another end of the inner tube (12) is defined with a communication port (121), one end of the outer tube (11) only communicates to the liquid inlet tube (13) and another end of the outer tube (11) is spaced apart from the communication port (121), and the liquid sequentially flows through the liquid inlet tube (13), the gap channel (S), the communication port (121), the inner tube (12), and the liquid outlet tube (14).

8. The fluid sterilization device (10) according to claim 7, wherein a portion of the liquid inlet tube (13) and a portion of the liquid outlet tube (14) each extend from two sides of one end of the outer tube (11), the main body (1) further comprises a first cover plate (16) and a second cover plate (17) that cover and seal two ends of the outer tube (11), the first cover plate (16) comprises a helical guide path (161), another portion of the liquid outlet tube (14), and a separator (164) positioned between the helical guide path (161) and the another portion of the liquid outlet tube (14), one end of the helical guide path (161) is arranged corresponding to the liquid inlet tube (13) and another end of the helical guide path (161) is arranged corresponding to the gap channel (S), and the helical guide path (161) comprises an opening (162) communicating with the gap channel (S) and a flow guide slope (163) that gradually narrows a distance with respect to the opening in a direction away from the liquid inlet tube (13).

9. The fluid sterilization device (10) according to claim 1, wherein the outer tube (11) and the inner tube (12) are both made of a reflective material, the main body (1) further comprises a liquid inlet tube (13) and a liquid outlet tube (14), one end of the outer tube (11) only communicates to the liquid inlet tube (13) and another end of the outer tube (11) only communicates to the liquid outlet tube (14), and the liquid sequentially flows through the liquid inlet tube (13), the gap channel (S), and the liquid outlet tube (14).

10. The fluid sterilization device (10) according to claim 9, wherein the liquid inlet tube (13) and the liquid outlet tube (14) extend from two ends of the outer tube (11), respectively, the main body (1) further comprises a first cover plate (16) and a second cover plate (17) that cover and seal two ends of the outer tube (11), the first cover plate (16) comprises a helical guide path (161), one end of the helical guide path (161) is arranged corresponding to the liquid inlet tube (13) and another end of the helical guide path (161) is arranged corresponding to the gap channel (S), and the helical guide path (161) comprises an opening (162) communicating with the gap channel (S) and a flow guide slope (163) that gradually narrows a distance with respect to the opening (162) in a direction away from the liquid inlet tube (13).
